# EUROPEAN PATENT APPLICATION

(11) **EP 1 462 792 A1**
(43) Date of publication of application: **29.09.2004**
(21) Application number: 03006924.9
(22) Date of filing: 26.03.2003
(51) Int. Cl.: G01N 21/35, G01N 21/95, G01N 33/15

(54) **Method and device for the analysis of products in the form of a capsule and of empty capsules by means of NIR reflection spectroscopy**

(71) Applicant: Pfizer GmbH Arzneimittelwerk Gödecke, 79090 Freiburg (DE)
(72) Inventor: Pysik, Alexander, 79194 Gundelfingen (DE); Haug, Holger Gerhard, 79106 Freiburg (DE)
(74) Representative: Tesch, Rudolf

(57) **Abstract**

A method and a device for the analysis of products in the form of a capsule and of empty capsules by means of NIR reflection spectroscopy are proposed in which the capsule is rotated around at least one axis during the recording of the reflection spectrum. A rotary drive (2) is provided on the capsule mounting (1) to this end.

## Description

The invention derives from a method and a device for the analysis of products in the form of a capsule and of empty capsules by means of NIR reflection spectroscopy.

NIR reflection spectroscopy is employed in pharmacy amongst others to analyse capsules and the content of capsules. It enables both the physical and chemical properties of the capsules to be determined. These include in particular the particle size, water content, identity or content of actives. Compared with chemical processes such as chromatography, NIR spectroscopy has the advantage that the sample in most cases does not have to be destroyed for the analysis and the gain of time because no or only minimal sample preparation is necessary.

The state of the art recognises methods and devices for NIR reflection spectroscopy on capsules in which the capsules are arranged at a fixed location. However, only a small part of the capsule case and contents can be measured when the capsules are in fixed positions. The distribution of the capsule contents in the capsule and the spread is not known in advance and is neither taken into consideration nor determined in the measurement. It may therefore be the case that, if the capsule is not completely full, the capsule contents are located only at one end of the capsule. If measurement is restricted to the middle of the capsule, the capsule contents are not captured in the measurement.

In contrast, the method according to the invention and the device according to the invention have the advantage that the rotation of the capsule during the measuring process ensures that several readings are obtained which yield a mean value in respect of the spread and density of the capsule contents. Should the capsule contents be only at one end of the capsule at the start of measurement, they are distributed over the entire capsule through the rotation. Non-reproducible conditions of the capsule contents in respect of the distribution within the capsule are thus balanced out. Furthermore, a large part of the capsule case can be captured with one measurement.

According to an advantageous configuration of the version, the capsule is arranged horizontally and rotated around its longitudinal axis. To this end the device has two jaws between which the capsule is fixed. At least one of the two jaws is brought into rotation by means of a rotary drive. This rotating movement is transmitted to the capsule and the second jaw. The rotation around the longitudinal axis of the horizontally arranged capsule has the advantage that the capsule contents are distributed over the entire capsule and do not slide from one end of the capsule to the other. The fixed location of the spectrometer also ensures that a large part of the capsule case is captured.

There is the further possibility that the capsule is arranged vertically or at any desired angle and that it is then rotated around axes perpendicular to the longitudinal axis.

In place of the reflection; the transmission of the NIR can also be determined and evaluated with the method according to the invention and the device according to the invention. The capsule mounting does not completely enclose the capsule, thereby allowing transmission of the NIR.

Further advantages and advantageous configurations of the invention can be obtained from the following description, the drawing and the claims.

### Drawings

The drawings show a model embodiment of a device according to the invention which is described in more detail below.
- Figure 1: Capsule mounting with rotary drive, perspectiv-partial cross-section view
- Figure 2: Pin mounting, front view

### Description of the embodiment of the invention

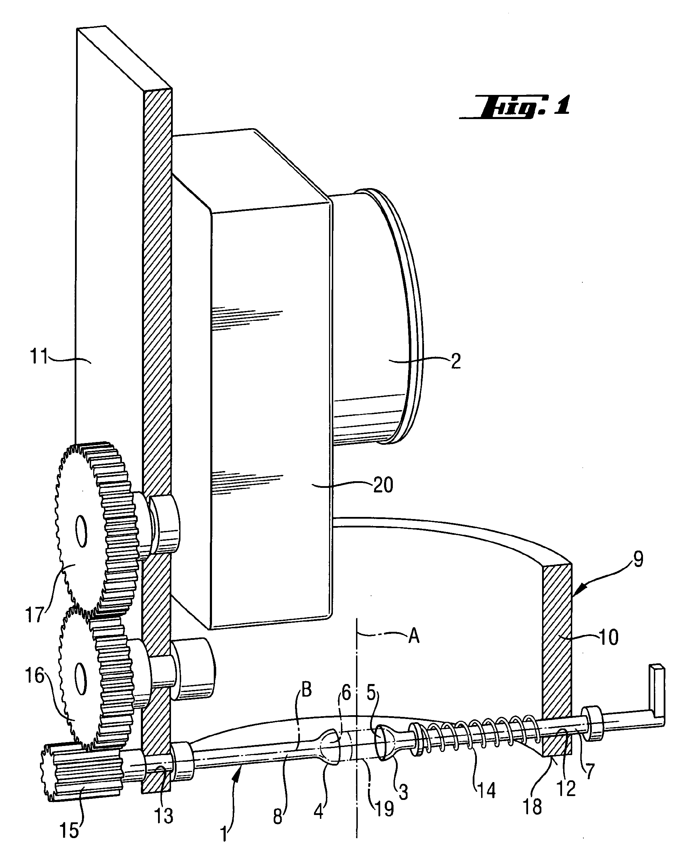

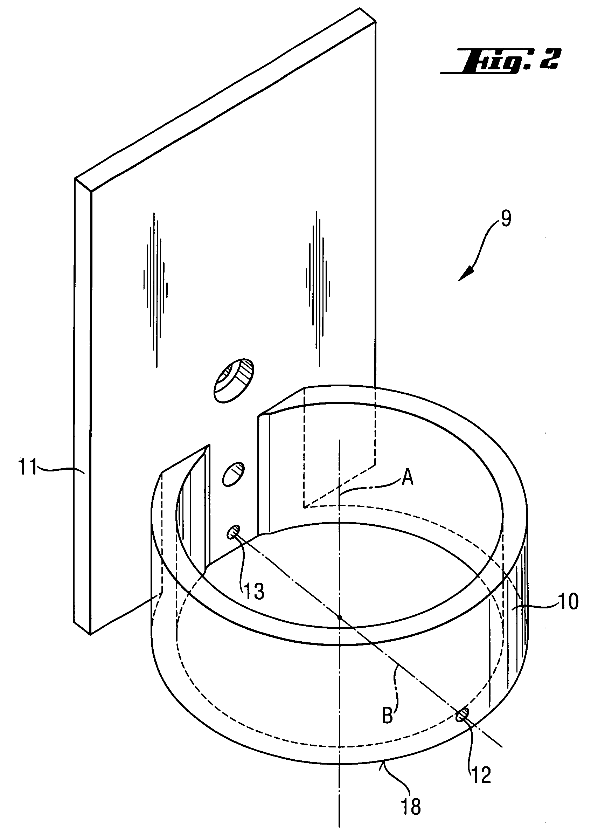

Figure 1 shows a capsule mounting 1 with rotary drive 2 in perspectiv-partial cross-section view. The capsule mounting essentially consists of two jaws 3 and 4 which have a hollow receptacle 5 and 6 for a capsule 19 schematically illustrated in the drawing. Each of the two jaws 3, 4 is secured to a pin 7, 8. The pin 7 is movably located in a pin mounting 9. The pin mounting 9 is shown in Figure 2 and consists of a tubular section 10 and a plate-shaped section 11. The tubular section 10 of the pin mounting 9 has recesses 12 and 13 adapted to the pins 7, 8 for the purpose of guiding the two cylindrical pins 7, 8. Around pin 7 is stretched a helical spring 14 which is fixed between the jaw 3 and the pin mounting 9. The helical spring 14 is pressed together by the clamping of a capsule 19 between the two jaws 3 and 4. The resulting spring force of the helical spring 14 ensures that the capsule 19 is clamped between the two jaws 3, 4. The pin 8 has a gear wheel 15 at the end projecting out of the pin mounting 9. The gear wheel 15 is connected to the rotary drive 2 by means of two further gear wheels 16 and 17. The gearing consisting of the gear wheels 15, 16 and 17 transmits the rotation of the rotary drive 2 to the pin 8 and thus to the capsule 19. The pin 7 is pivoted in the pin mounting 9 and is moved by the rotation of the capsule 19. A suitable rotary drive 2 would be an electric motor with gear box 20, for instance. However, there is also the possibility of initiating the rotation manually by means of a handle or a crank not shown in the drawing.

The capsule mounting is placed with its front side 18 on an NIR spectrometer for the purpose of conducting the measurements. The beam axis A of the NIR runs perpendicular to the longitudinal axis B of the capsule 19. Since the two jaws 3 and 4 cover only a small part of the capsule, almost the entire capsule is captured by the NIR. Because of the rotation of the capsule 19 around its longitudinal axis B, almost the entire surface of the capsule and the entire capsule contents can be analysed.

All features of the invention can be material to the invention both individually and in any combination.

### Reference numbers

- 1: Capsule mounting
- 2: Rotary drive
- 3: Jaw
- 4: Jaw
- 5: Receptacle for the capsule
- 6: Receptacle for the capsule
- 7: Pin
- 8: Pin
- 9: Pin mounting
- 10: Tubular section of the pin mounting
- 11: Plate-shaped section of the pin mounting
- 12: Recess for pin
- 13: Recess for pin
- 14: Helical spring
- 15: Gear wheel
- 16: Gear wheel
- 17: Gear wheel
- 18: Front side
- 19: Capsule
- 20: Gear box
- A: Beam axis of NIR
- B: Longitudinal axis of capsule 19 and capsule mounting 1

## Claims

1. Method for the analysis of products in the form of a capsule and of empty capsules by means of NIR reflection spectroscopy, **characterised in that** the capsule is rotated around at least one axis during the recording of the reflection spectrum.

2. Method according to claim 1, **characterised in that** the capsule is arranged horizontally and rotated around its longitudinal axis.

3. Device for the analysis of products in the form of a capsule and of empty capsules by means of NIR reflection spectroscopy, in particular for the performance of the method according to claim 1 and 2,
with an NIR spectrometer and
with a capsule mounting for the capsule,
**characterised in that**
the capsule mounting is pivoted and a rotary drive is provided for the rotation of the capsule mounting around at least one axis.

4. Device according to claim 3, **characterised in that** two jaws are provided as a capsule mounting with in each case a cavity facing the capsule in the mounted state for the accommodation of one end of the capsule.

5. Device according to claim 4, **characterised in that** a spring is provided which presses the two jaws together.

6. Device according to claim 4 or 5, **characterised in that** each of the two jaws is arranged on a pin.

7. Device according to claim 6, **characterised in that** a pin mounting is provided in which one of the two pins is axially movably mounted and that a helical spring fixed between one of the two jaws and the mounting is provided as the spring.

8. Device according to claim 7, **characterised in that** the rotary drive is connected with at least one of the two pins.

9. Device according to claim 8, **characterised in that** the rotary drive is connected with at least one of the two pins by means of a toothed gearing.
